# EUROPEAN PATENT APPLICATION

(11) **EP 1 170 389 A2**
(43) Date of publication of application: **09.01.2002**
(21) Application number: 01116257.5
(22) Date of filing: 30.01.1997
(51) Int. Cl.: C22B 3/26, C22B 15/00, C07C 49/12

(54) **Improved beta-diketones for the extraction of copper from aqueous ammoniacal solutions**

(30) Priority: 06.02.1996 US 11207; 08.01.1997 US 780759
(62) Divisional of application: 97904756.0
(71) Applicant: HENKEL CORPORATION, Gulph Mills, Pennsylvania 19406 (US)
(72) Inventor: Virnig, Michael J., Tucson, AZ 85745 (US); Kordosky, Gary A., Tucson, AZ 85745 (US)
(74) Representative: Weber, Thomas, Dr. Dipl.-Chem.

(57) **Abstract**

A process for recovery of copper from an aqueous ammoniacal solution containing copper values comprising:
(A) contacting a copper pregnant aqueous ammoniacal solution containing copper values with a water insoluble beta-diketone copper extractant dissolved in a water-immiscible organic solvent to extract copper values from said aqueous ammoniacal solution into said organic solution thereby forming a copper pregnant organic phase and a copper depleted aqueous phase;
(B) separating said aqueous phase and said organic phase;
(C) contacting the copper pregnant organic phase with an aqueous acidic stripping solution;
(D) separating said aqueous acidic stripping solution now containing the copper values from the organic phase; and
(E) recovering the copper from said aqueous acidic stripping solution; characterized in that the beta-diketone extractant being a highly sterically hindered beta-diketone selected from beta-diketones of formula II: 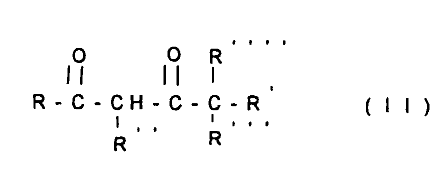 where R is phenyl or alkyl substituted phenyl, R' is alkyl having from above 1 to 8 carbon atoms, and R", R"' and R"" are the same or different and are chosen from the group consisting of H, alkyl having from 1 to 8 carbon atoms, and aralkyl having from 7 to 14 carbon atoms, with the proviso that (a) less than two of R", R"' and R"" are H and (b) the total number of carbons in all R groups is at least 13.

## Description

### Field of the invention

This invention relates to new, improved beta-diketones and their use in the extraction of copper from aqueous ammoniacal solutions containing copper values, resulting from commercial processes, including, but not limited to, leaching of copper containing ores, such as sulfidic ores, or concentrates resulting from flotation of such sulfidic ores.

### Statement of Related Art

Practice in the recovery of copper from its sulfidic ores involves subjecting the ores to a froth flotation operation to produce a concentrate of the valuable metal sulfides and to reject the flotation tailings of valueless sulfides, silicates, aluminates and the like. One of such concentrates provided is a chalcocite concentrate containing chalcocite and covellite.

In US Patent 4,022,866 to Kuhn and Arbiter, and in their subsequent paper, "Physical and Chemical Separations via the Arbiter process" 11th International Mining Congress, April, 1975, Cagliari, Italy; proc.-Int. Miner. Process. Congress., paper 30; pp.831-847; there is described the leaching of copper sulfide concentrates with ammonia/ammonium sulfate and oxygen whereby the sulfate, and the dissolved copper may then be recovered by solvent extraction. The solvent extraction reagents are described in the patent only generally as those which preferentially load copper from alkaline solutions. In the paper, which describes the Arbiter process, the focus is on complete or nearly complete leaching of the concentrate. Another Kuhn and Arbiter paper, "Anaconda's Arbiter Process for Copper", Hydrometallurgy, CIM Bulletin, Feb, 1974, contains similar description.

U.S. Patent 4,563,256 describes a solvent extraction process for the recovery of zinc values from ammoniacal solutions, which may also contain copper values, using or employing various oxime extractants.

U.S. Patent 2,727.818 describes a method of leaching copper sulfide materials with ammoniacal leach solutions. No solvent extraction is discussed.

U.S. Patents 4,065,502 and 4,175,012 describes beta-diketones which may be employed as metal extractants in a liquid-liquid ion exchange process for recovery of metals, such as nickel or copper, from aqueous solutions containing the metal values, including aqueous ammoniacal solutions.

U.S. Patent 4,350,661 describes the extraction of copper from ammoniacal aqueous solutions by a process of extraction first with a beta-diketone followed by a second extraction with an oxime. Alternatively, there is described the use of a mixture of diketone and oxime wherein the extractant reagent comprises about 5-30 percent by volume of the strong reagent (oxime) and 10-60 percent by volume of the weak reagent (beta diketone).

In commonly assigned, co-pending application, U.S. Serial No. 07/745,028, the entire disclosure of which is hereby incorporated by reference, there is described a partial leaching of a chalcocite concentrate, to provide an aqueous ammoniacal leach solution containing copper values, and the use of high copper transfer, low ammonia loading extraction reagents such as a beta-diketone. An oxime extractant is also disclosed alone or with the diketone.

### DESCRIPTION OF THE INVENTION

In this description, except in the operating examples or where explicitly otherwise indicated, all numbers describing amounts of ingredients or reaction conditions are to be understood as modified by the word "about".

It was found that when contacted with an ammoniacal solution obtained by leaching a copper sulfide concentrate with ammonia/ammonium sulfate, a preferred diketone extractant reagent, 1-phenyl-3-isoheptyl-1,3-propanedione, will extract the copper but upon repetitive use the copper may become more difficult to strip. It is believed that the problem may arise from a synergistic interaction of surfactant type materials introduced into the organic phase through a leach liquor, with a ketimine that is formed by reaction of ammonia with the beta-diketone.

It has now been discovered that the use of a sterically hindered beta-diketone, will provide a very efficient and improved process for the recovery of copper in that a significant improvement in the stability of strip kinetics will be achieved. It is believed that the steric hindrance around the beta-diketone functionality results in more stability to use conditions, minimizing, if not eliminating, ketimine formation.

By "highly sterically hindered diketone" as employed herein is meant that the diketone is highly sterically hindered where the three carbons attached to the carbonyl carbons of the diketone can together bear no more than four hydrogens when none of the three carbons is a part of a phenyl ring and no more than three hydrogens when one of the three carbons is part of a phenyl ring. It is also required that the carbon atom between the two carbonyl carbons must bear at least one hydrogen.

While the present invention is particularly useful in applications where ammoniacal leach solutions are encountered in the treatment of copper containing sulfidic ores, the present invention is applicable or useful in the extraction of copper from any aqueous ammoniacal solution containing copper values regardless of its source.

In its general application accordingly the present invention is an improvement in the process of extraction and recovery of copper from aqueous ammoniacal solutions in a process comprising:
(1) contacting a copper pregnant aqueous ammoniacal solution containing copper values with a water insoluble beta-diketone copper extractant dissolved in a water immiscible organic solvent to extract copper values from said aqueous ammoniacal solution into said organic solution thereby forming a copper pregnant organic phase and a copper depleted aqueous phase;
(2) separating said aqueous phase and said organic phase;
(3) contacting the copper pregnant organic phase with an aqueous acidic stripping solution, whereby copper values are stripped from the organic phase into the aqueous acidic stripping solution;
(4) separating said aqueous acidic stripping solution from said organic phase; and
(5) recovering the copper from said aqueous acidic stripping solution; the improvement in the process in the present invention being the use of a highly sterically hindered beta-diketone extractant.

In view thereof, the present invention also contemplates a new copper extractant, which comprises a beta-diketone copper extractant having an alkyl or aralkyl substituent attached to the carbon atom which joins the two carbonyl groups of the diketone, such as 1-phenyl-2-methyl or benzyl-3-isoheptyl-1,3-propanedione, or the beta-diketone extractant, 1-phenyl-3-(1-ethylpentyl)-1,3-propanedione.

As indicated earlier, the present invention is useful in regard to aqueous ammoniacal solutions from a variety of sources such a those encountered in leaching of chalcocite concentrates. In such applications the copper pregnant leach solutions from which the copper is to be recovered by extraction will contain on the order of about 15-170 g/l copper and typically about 30-40 g/l copper at a pH of about 8.5 to 11. In solutions encountered from other applications, the solutions may contain copper at higher levels, on the order of 125-170 g/l, such as solutions encountered in ammoniacal copper chloride printed board etchants.

The improvement in the process comprises employing a sterically hindered beta-diketone of the formula I or II. The sterically hindered beta-diketones may be of the structure [I]: 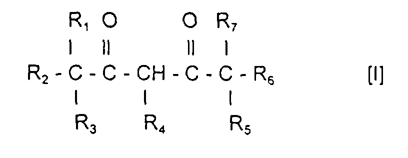 wherein R₁ through R₇ are the same or different, and are chosen from the group consisting of hydrogen, an aryl radical, an alkaryl radical containing from 6 to about 18 carbon atoms, and an alkyl radical containing from 1 to about 13 carbon atoms, with the provisos that (a) any two of R₁ through R₇ may together form a carbocyclic ring, (b) no more than four of R₁ through R₇ may be hydrogen, and (c) the overall molecule contains at least 14 carbon atoms.

In a second embodiment of the invention, the sterically hindered beta-diketones may be of the formula [II]: 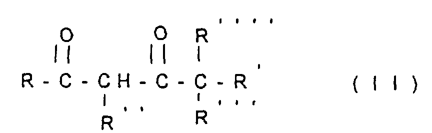 Compounds of formula II are modifications of the beta-diketones found in U.S. Patent 4,065,502 and 4,175,012. The hindered beta-diketones of the present invention are those where R is phenyl or alkyl substituted phenyl, R' is alkyl, and R", R"' and R"" are the same or different and are chosen from the group consisting of H, alkyl having from 1 to about 8 carbon atoms, and aralkyl having from 7 to about 14 carbon atoms, with the proviso that (a) no more than two of R", R"' and R"" are H and (b) the total number of carbons in all R groups is at least 13. Preferred compounds are those in which (i) R is phenyl, R" is methyl or benzyl, R' is branched hexyl, and R"' and R"" are H; (ii) R is phenyl, R" is H, R' is butyl, R"' is ethyl and R"" is H, and (iii) R is phenyl, R" is H, R' is a straight or branched chain alkyl group containing from 5-8 carbon atoms and R"' and R"" are methyl.

The various R groups in formula I or II are preferably free from substitution and each contains less than about 20 carbon atoms.

The preferred diketone found to be particularly suitable in the past is 1-phenyl-3-isoheptyl-1,3-propanedione wherein the carbon attaching the isoheptyl group to the carbonyl group is not tertiary. However, as earlier noted, when such diketone under conditions of use is exposed to high levels of ammonia, and particularly where relatively high temperatures are encountered, such as 45 degrees Centigrade, the result is the formation of the corresponding ketimine which then results in poor stripping and may be an intermediate in a degradation pathway resulting in by-products that may contribute to very high entrainment of the aqueous phase in the loaded organic.

In the process of extraction a wide variety of water immiscible liquid hydrocarbon solvents can be used in the copper recovery process to form the organic phase in which the diketone extractant is dissolved. These include aliphatic and aromatic hydrocarbons such as kerosenes, benzene, toluene, xylene and the like. A choice of essentially water-immiscible hydrocarbon solvents or mixtures thereof will depend on factors, including the plant design of the solvent extraction plant, (mixer-settler units, extractors) and the like. The preferred solvents for use in the present invention are the aliphatic or aromatic hydrocarbons having flash points of 130 degrees Fahrenheit and higher, preferably at least 150 degrees and solubilities in water of less than 0.1 % by weight. The solvents are essentially chemically inert. Representative commercially available solvents are Chevron™ ion exchange solvent (available from Standard Oil of California) having a flash point of 195 degrees Fahrenheit; Escaid™ 100 and 110 (available from Exxon-Europe) having a flash point of 180 degrees Fahrenheit; Norpar™ 12 (available from Exxon-USA) with a flash point of 160 degrees Fahrenheit; Conoco™ C1214 (available from Conoco) with a flash point of 160 degrees Fahrenheit; and Aromatic 150 (an aromatic kerosene available from Exxon-USA having a flash point of 150 degrees Fahrenheit), and other various kerosenes and petroleum fractions available from other oil companies.

In the extraction process, the organic solvent solutions may contain the beta-diketone in an amount approaching 100 % solids, but typically the diketone will be employed in an amount of about 20-30% by weight.

In the process, the volume ratios of organic to aqueous (O:A) phase will vary widely since the contacting of any quantity of the diketone organic solution with the copper containing aqueous ammoniacal solution will result in the extraction of copper values into the organic phase. For commercial practicality however, the organic:aqueous phase ratios for extraction are preferably in the range of about 50:1 to 1:50. It is desirable to maintain an effective O:A ratio of about 1:1 in the mixer unit by recycle of one of the streams. In the stripping step, the organic:aqueous stripping medium phase will preferably be in the range of about 1:4 to 20:1. For practical purposes, the extracting and stripping are normally conducted at ambient temperatures and pressure although higher and lower temperatures and pressures are entirely operable. While the entire operation can be carried out as a batch operation, most advantageously the process is carried out continuously with the various streams or solutions being recycled to the various operations in the process for recovery of the copper, including the leaching, extraction and the stripping steps.

In the extraction process the extractant reagent should be soluble in the organic water-immiscible solvent. In general the diketones of the present invention will be soluble to such an extent and amount described above. If necessary or desirable to promote specific desired properties of extraction, solubility modifiers generally known in the art may be employed. Such modifiers include long chain (6-30 carbon aliphatic alcohols or esters such as n-hexanol, n-2-ethylhexanol, isodecanol, isohexadecanol, 2-(1,3,3 trimethylbutyl)-5,7,7-trimethyl octanol and 2,2,4-trimethyl-1,3-pentanediol mono-or di- isobutyrate, long chain phenols, such as heptylphenol, octylphenol, nonylphenol and dodecylphenol; and organo phosphorous compounds such as tri-lower alkyl (4-8 carbon atom) phosphates especially tributyl phosphate and tri-(2-ethylhexyl) phosphate. Where indicated to be desirable, kinetic additives may also be employed. It is preferred to avoid solubility modifiers.

The present invention also contemplates the use of a catalytic amount of an oxime, in which case the oxime functions as a kinetic additive, in combination with the sterically hindered diketones of the present invention, preferably an hydroxy, aryl oxime. By catalytic amount as employed herein, is meant a small amount of oxime in relation to the amount of diketone, preferably from about 0.5 to about 5 mole % of oxime relative to the beta-diketone. Such amounts of oxime are particularly effective in accelerating the rate of copper stripping from the organic phase.

The beta-diketones of this invention are also effective as co-extractants for copper from ammoniacal solutions along with strong extractants, such as oximes, a combination similar to that described in US Patent 4,350,661 noted earlier above. In such a case, the mixture of diketone and oxime in the extraction reagent will comprise about 5-30 volume percent of the oxime strong reagent and about 10-60 volume percent of the weaker beta-diketone.

The oxime compounds which are to be employed in catalytic amounts along with the sterically hindered beta-diketones, or may be co-extractants with the beta-diketones are certain oximes such as described in US Patent 4,563.256. Such oximes, which may be employed are those generally conforming to the formula: 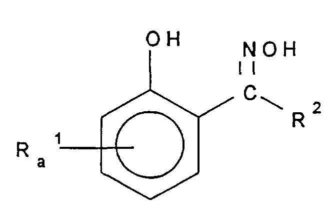 where R¹ is a saturated aliphatic group of 1-25 carbon atoms or an ethylenically unsaturated group of 3-25 carbon atoms or OR³, where R³ is a saturated or ethylenically unsaturated group as defined above, a is an integer of 0, 1, 2, 3 or 4 and R² is H, or a saturated or ethylenically unsaturated group as defined above, with the proviso that the total number of carbon atoms in R¹ and R² is from 3-25, or is phenyl or R⁴ substituted phenyl where R⁴ is a saturated or ethylenically unsaturated group as defined above, which may be the same or different from R¹. Illustrative of some of the oxime compounds are 5-heptyl salicylaldoxime, 5-octyl salicylaldoxime, 5-nonyl salicylaldoxime, 5-dodecyl salicylaldoxime, 5-nonyl-2-hydroxyacetophenone oxime, 5-dodecyl-2-hydroxyacetophenone oxime, 2-hydroxy-5-nonyl benzophenone oxime and 2-hydroxy-5-dodecyl benzophenone oxime. While it may be preferred that a single oxime compound be employed along with the beta-diketone, mixtures of oximes may be employed to meet particular system requirements.

In the stripping step, a sulfuric acid solution containing about 60-180 g/l sulfuric acid is the preferred stripping agent as it permits the subsequent recovery of the copper by conventional recovery steps either in the form of copper sulphate crystals or by electrowinning to cathode copper. Other mineral acids may be used such as hydrochloric or nitric; however, such may require other recovery methods or specialized handling equipment.

The following example is illustrative of the procedure by which an alkyl or aralkyl substituent may be introduced into the beta-diketones of Mackay U.S. Patents 4,065,502 and 4,175,012, such as 1-phenyl-3-isoheptyl-1,3-propanedione.

A mixture of 55.2 g anhydrous potassium carbonate, 1.6 g tetrabutylammonium fluoride, 24.8 g 1-phenyl-3-isoheptyl-1,3-propanedione and 150 ml. toluene were stirred and heated to reflux for 2 hours with a Dean-Stark trap under a nitrogen atmosphere. The mixture was cooled to room temperature, 7.0 ml methyl iodide was added and the mixture was heated with stirring to 50 degrees Centigrade overnight. The product was diluted with toluene and water, and the organic phase washed with water, and stripped under reduced pressure. The residue was distilled in a Kugelrohr apparatus at 100-110 degrees Centigrade at 0.3 mm Hg to give 25.6 g distillate. The distillate was purified on a silica gel column and redistilled in a Kugelrohr apparatus to give 16.4g of 1-phenyl-2-methyl-3-isoheptyl-1,3-propanedione. Employing the same procedure using benzyl iodide will provide the corresponding 1-phenyl-2-benzyl-3-isoheptyl-1,3-propanedione.

Other hindered beta-diketones may be prepared by the method of Example A of U.S. Patent 4,065,502. Thus, methyl 2-ethylhexanoate is condensed with acetophenone to give 1-phenyl-4-ethyl-1,3-nonanedione, and methyl 2,2-dimethyl octanoate is condensed with acetophenone to give 1-phenyl-4,4-dimethyl-1,3-undecanedione.

These hindered beta-diketones are then employed in a process for the recovery and extraction of copper from an aqueous ammoniacal copper containing solution as described earlier above in steps (1) through (5).

In a process for recovery of copper from an aqueous ammoniacal solution containing copper values comprising:
(A) contacting a copper pregnant aqueous ammoniacal solution containing copper values with a water insoluble beta-diketone copper extractant dissolved in a water-immiscible organic solvent to extract copper values from said aqueous ammoniacal solution into said organic solution thereby forming a copper pregnant organic phase and a copper depleted aqueous phase;
(B) separating said aqueous phase and said organic phase;
(C) contacting the copper pregnant organic phase with an aqueous acidic stripping solution;
(D) separating said aqueous acidic stripping solution now containing the copper values from the organic phase; and
(E) recovering the copper from said aqueous acidic stripping solution;
wherein the improvement comprises said water insoluble
beta-diketone extractant being a highly sterically hindered beta-diketone.

A process as defined above wherein the sterically hindered beta-diketone has the formula I: 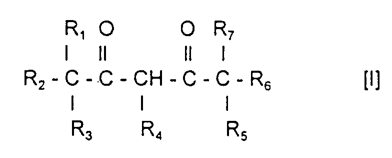 wherein R₁ through R₇ are the same or different, and are chosen from the group consisting of hydrogen, an aryl radical, an alkaryl radical containing from 6 to about 18 carbon atoms, and an alkyl radical containing from 1 to about 13 carbon atoms, with the provisos that (a) any two of R₁ through R₇ may together form a carbocyclic ring, (b) no more than four of R₁ through R₇ may be hydrogen, and (c) the overall molecule contains at least 14 carbon atoms.

A process as defined above, wherein the sterically hindered beta-diketone has the formula II: 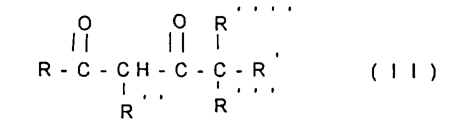 where R is phenyl or alkyl substituted phenyl, R' is alkyl, and R", R"' and R"" are the same or different and are chosen from the group consisting of H, alkyl having from 1 to about 8 carbon atoms, and aralkyl having from 7 to about 14 carbon atoms, with the proviso that (a) no more than two of R", R"' and R"" are H and (b) the total number of carbons in all R groups is at least 13.

A process as defined above, wherein R is phenyl, R" is methyl or benzyl, R' is branched hexyl and R"' and R"" are H.

A process as defined above wherein R is phenyl, R" is H, R' is butyl, R"' is ethyl and R"" is H.

A process as defined above, wherein R is phenyl, R" is H, R' is a branched chain alkyl group having from 5-8 carbon atoms and R"' and R"" are methyl.

A process as defined above, wherein the sterically hindered beta-diketone is one in which an alkyl or aralkyl substituent having from 1 to 7 carbon atoms is attached to the carbon atom joining the two carbonyl groups of the beta-diketone.

A process as defined above wherein R" is selected from the group consisting of methyl and benzyl.

A process as defined above wherein the diketone extractant is 1-phenyl-2-methyl-3-isoheptyl-1,3-propanedione.

A process as defined above wherein the diketone extractant is 1-phenyl-2-benzyl-3-isoheptyl-1,3-propanedione.

A process as defined above wherein the diketone is 1-phenyl-4-ethyl-1,3-nonanedione.

A process as defined above wherein
the diketone is 1-phenyl-4,4-dimethyl-1,3-undecanedione.

A process as defined in claim 1, in which the extractant further comprises an hydroxy aryl oxime.

A process as defined above, wherein said hydroxy aryl oxime is present in a catalytic amount.

A process as defined above, wherein said hydroxyaryl oxime is a co-extractant with the beta-diketone.

A process as defined above, wherein said hydroxy aryl oxime has the formula: 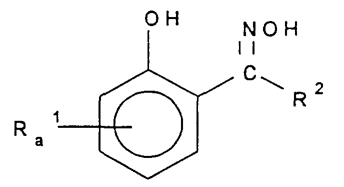 where R¹ is a saturated aliphatic group of 1-25 carbon atoms or an ethylenically unsaturated group of 3-25 carbon atoms or OR³, where R³ is a saturated or ethylenically unsaturated group as defined above, a is an integer of 0, 1, 2, 3 or 4 and R² is H, or a saturated or ethylenically unsaturated group as defined above, with the proviso that the total number of carbon atoms in R¹ and R² is from 3-25, or is phenyl or R⁴ substituted phenyl where R⁴ is a saturated or ethylenically unsaturated group as defined above, which may be the same or different from R¹.

A process as defined above, in which said hydroxy aryl oxime is selected from the group consisting of 5-heptyl salicylaldoxime, 5-octyl salicylaldoxime, 5-nonyl salicylaldoxime, 5-dodecyl salicylaldoxime, 5-nonyl-2-hydroxyacetophenone oxime, 5-dodecyl-2-hydroxyacetophenone oxime, 2-hydroxy-5-nonyl benzophenone oxime and 2-hydroxy-5-dodecyl benzophenone oxime.

A beta-diketone extractant compound adapted for extracting copper values from aqueous ammoniacal copper containing solutions comprising a beta-diketone compound of the formula II: 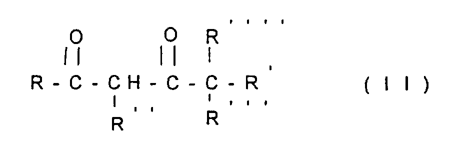 where R is phenyl or alkyl substituted phenyl, R' is alkyl, and R", R"' and R"" are the same or different and are chosen from the group consisting of H, alkyl having from 1 to about 8 carbon atoms, and aralkyl having from 7 to about 14 carbon atoms, with the proviso that (a) no more than two of R", R"' and R"" are H and (b) the total number of carbons in all R groups is at least 13.

A beta-diketone compound as defined above, in which R" is selected from the group consisting of methyl and benzyl.

A beta-diketone compound as defined above, wherein R is phenyl, R" is methyl or benzyl, R' is branched hexyl and R"' and R"" are H.

A beta-diketone compound as defined above, wherein R is phenyl, R" is H, R' is butyl, R"' is ethyl and R"" is H.

A beta-diketone compound as defined above, wherein R is phenyl, R" is H, R' is a branched chain alkyl group having from 5-8 carbon atoms and R"' and R"" are methyl.

1-phenyl-2-benzyl-3-isoheptyl-1,3-propanedione.

1-phenyl-2-methyl-3-isoheptyl-1,3-propanedione.

1-phenyl-4-ethyl-1,3-nonanedione

1-phenyl-4,4-dimethyl-1,3-undecanedione.

## Claims

1. A process for recovery of copper from an aqueous ammoniacal solution containing copper values comprising:
(A) contacting a copper pregnant aqueous ammoniacal solution containing copper values with a water insoluble beta-diketone copper extractant dissolved in a water-immiscible organic solvent to extract copper values from said aqueous ammoniacal solution into said organic solution thereby forming a copper pregnant organic phase and a copper depleted aqueous phase;
(B) separating said aqueous phase and said organic phase;
(C) contacting the copper pregnant organic phase with an aqueous acidic stripping solution;
(D) separating said aqueous acidic stripping solution now containing the copper values from the organic phase; and
(E) recovering the copper from said aqueous acidic stripping solution; **characterized in that** the beta-diketone extractant being a highly sterically hindered beta-diketone selected from beta-diketones of formula II: 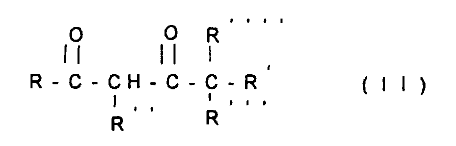 where R is phenyl or alkyl substituted phenyl, R' is alkyl having from above 1 to 8 carbon atoms, and R", R"' and R"" are the same or different and are chosen from the group consisting of H, alkyl having from 1 to 8 carbon atoms, and aralkyl having from 7 to 14 carbon atoms, with the proviso that (a) less than two of R", R"' and R"" are H and (b) the total number of carbons in all R groups is at least 13.

2. A process as defined in claim 1, wherein R is phenyl, R" is H, R' is a branched chain alkyl group having from 5-8 carbon atoms and R"' and R"" are methyl.

3. A process as defined in claim 1 wherein R" is selected from the group consisting of methyl and benzyl.

4. A process as defined in claim 1 wherein the diketone extractant is 1-phenyl-2-methyl-3-isoheptyl-1,3-propanedione.

5. A process as defined in claim 1 wherein the diketone extractant is 1-phenyl-2-benzyl-3-isoheptyl-1,3-propanedione.

6. A process as defined in claim 1 wherein the diketone is 1-phenyl-4,4-dimethyl-1,3-undecanedione.

7. A process as defined in claim 1, in which the extractant further comprises an hydroxy aryl oxime.

8. A process as defined in claim 7, wherein said hydroxy aryl oxime is present in a catalytic amount.

9. A process as defined in claim 7, wherein said hydroxyaryl oxime is a co-extractant with the beta-diketone.

10. A process as defined in claim 7, wherein said hydroxy aryl oxime has the formula: 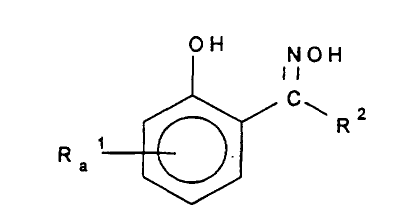 where R' is a saturated aliphatic group of 1-25 carbon atoms or an ethylenically unsaturated group of 3-25 carbon atoms or OR³, where R³ is a saturated or ethylenically unsaturated group as defined above, a is an integer of 0, 1, 2, 3 or 4 and R² is H, or a saturated or ethylenically unsaturated group as defined above, with the proviso that the total number of carbon atoms in R¹ and R² is from 3-25, or is phenyl or R⁴ substituted phenyl where R⁴ is a saturated or ethylenically unsaturated group as defined above, which may be the saure or different from R¹.

11. A process as defined in claim 10, in which said hydroxy aryl oxime is selected from the group consisting of 5-heptyl salicylaldoxime, 5-octyl salicylaldoxime, 5-nonyl salicylaldoxime, 5-dodecyl salicylaldoxime, 5-nonyl-2-hydroxyacetophenone oxime, 5-dodecyl-2-hydroxyacetophenone oxime, 2-hydroxy-5-nonyl benzophenone oxime and 2-hydroxy-5-dodecyl benzophenone oxime.

12. A beta-diketone extractant compound adapted for extracting copper values from aqueous ammoniacal copper containing solutions comprising a beta diketone compound of the formula II: 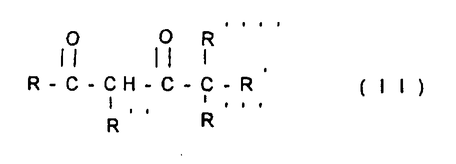 where R is phenyl or alkyl substituted phenyl, R' is alkyl having from above 1 to 8 carbon atoms, and R", R"' and R"" are the same or different and are chosen from the group consisting of H, alkyl having from 1 to 8 carbon atoms, and aralkyl having from 7 to 14 carbon atoms, with the proviso that (a) less than two of R", R"' and R"" are H and (b) the total number of carbons in all R groups is at least 13.

13. A beta-diketone compound as defined in claim 12, in which R" is selected from the group consisting of methyl and benzyl.

14. A beta-diketone compound as defined in claim 12, wherein R is phenyl, R" is methyl or benzyl, R' is branched hexyl and R"' and R"" are H.

15. A beta-diketone compound as defined in claim 12, wherein R is phenyl, R" is H, R' is a branched chain alkyl group having from 5-8 carbon atoms and R"' and R"" are methyl.

16. A beta diketone according to claim 12 selected from 1-phenyl-2-benzyl-3-isoheptyl-1,3-propanedione, 1-phenyl-2-methyl-3-isoheptyl-1,3-propanedione and 1-phenyl-4,4-dimethyl-1,3-undecanedione.
